(19) Europäisches Patentamt
European Patent Office
Office européen des brevets

(11) Publication number: 0 057 514
B1

(12) EUROPEAN PATENT SPECIFICATION

(45) Date of publication of patent specification: 10.04.85

(51) Int. Cl.$^4$: C 07 C 2/86, C 07 C 4/12

(21) Application number: 82300164.9

(22) Date of filing: 13.01.82

# (54) Process for alkylating aromatic compounds.

(30) Priority: 30.01.81 US 230176

(43) Date of publication of application:
11.08.82 Bulletin 82/32

(45) Publication of the grant of the patent:
10.04.85 Bulletin 85/15

(84) Designated Contracting States:
BE DE FR GB IT NL

(56) References cited:
EP-A-0 012 569
EP-A-0 014 545
EP-A-0 018 117
US-A-3 718 704
US-A-4 086 289
US-A-4 101 595

(73) Proprietor: MOBIL OIL CORPORATION
150 East 42nd Street
New York New York 10017 (US)

(72) Inventor: Haag, Werner Otto
38 Pine Knoll Drive
Lawrenceville New Jersey (US)
Inventor: Huang, Tracy Jau-Hua
9 Wood Field Lane
Lawrenceville New Jersey (US)

(74) Representative: West, Alan Harry et al
Mobil Court 3 Clements Inn
London WC2A 2EB (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 057 514 B1

Courier Press, Leamington Spa, England.

**Description**

This invention relates to the alkylation of aromatic compounds.

The alkylation of aromatic hydrocarbons utilizing crystalline aluminosilicate catalysts has already been described. U.S. Patent 3,251,897 for example, describes the alkylation of aromatic hydrocarbons in the presence of X- or Y-type crystalline aluminosilicate zeolites, specifically those in which the cation is rare earth and/or hydrogen. U.S. Patents 3,751,504 and 3,751,506 describe the vapor phase alkylation of aromatic hydrocarbons with olefins, for example of benzene with ethylene, in the presence of a ZSM-5 type zeolite catalyst.

Processes for the catalytic methylation of aromatic hydrocarbons, for example of toluene to provide a mixture of xylene isomers, employing hydrogen and carbon monoxide as the methylating agent are described in U.S. Patents 3,718,704 and 4,086,289. Due to the nature of the catalysts used in those processes, namely a mixture of certain metal oxides such as zinc oxide and chromia (zinc chromite) and zinc chromite mixed with an alkali metal-exchanged molecular sieve containing an excess of alkali metal carbonate, respectively, those processes are incapable of incorporating alkyl groups other than methyl groups into the aromatic ring.

The present invention is based on the observation that alkyl side chains having two or more carbon atoms can be incorporated into aromatic compounds using hydrogen/hydrogen precursor and carbon oxide mixtures as the alkylating agent over certain heterogeneous catalysts, without substantial amounts of methyl aromatics being produced simultaneously. Such a process has the most distinct advantage that the resulting $C_2$+ alkylaromatics can be used for olefin production by subjecting them to dealkylation.

According to the present invention, there is provided a process for alkylating an aromatic compound which comprises reacting at least one aromatic compound having at least one hydrogen atom directly bonded to the aromatic nucleus with an alkylating agent comprising a mixture of hydrogen and/or a hydrogen-containing substance which provides hydrogen under the process conditions, and a carbon oxide, at an elevated temperature in the presence of a catalyst, characterized in that the catalyst comprises a metal or metal-containing component selected from iron, cobalt, ruthenium, manganese, rhodium and osmium, and a zeolite having a Constraint Index of not more than 12, and recovering a product comprising an aromatic compound having at least one more alkyl group of at least two carbon atoms than the starting aromatic compound.

In addition to providing an economical one-step synthesis for the production of industrially important $C_2$+ alkyl aromatics, the alkylation process of the invention also offers a practical and efficient first step in the production of olefins which may be obtained by dealkylation of the product alkyl aromatics employing known and conventional methods. The dealkylated aromatics may then be recycled to the alkylation reaction for conversion to further quantities of alkyl aromatics. These reactions, alkylation and dealkylation, can be illustrated for the preferred alkylating agent, synthesis gas or "syngas" which is a gaseous mixture containing hydrogen and carbon monoxide obtained from the gasification of carbonaceous materials such as coal or natural gas, as follows (Ar is aryl and n=2 to about 22):

$$ArH+2nH_2+nCO \xrightarrow{\text{alkylation}} ArC_nH_{2n+1}+nH_2O$$

$$ArC_nH_{2n+1} \xrightarrow{\text{dealkylation}} ArH+C_nH_{2n}$$

or

$$ArH+nH_2+2nCO \xrightarrow{\text{alkylation}} ArC_nH_{2n+1}+nCO_2$$

$$ArC_nH_{2n+1} \xrightarrow{\text{dealkylation}} ArH+C_nH_{2n}$$

With recycle of the dealkylated aromatic compound, it can be seen from the above that the net reaction providing olefin consumes only syngas as indicated by

$$2nH_2+nCO \rightarrow C_nH_{2n}+nH_2O$$

or

$$nH_2+2nCO \rightarrow C_nH_{2n}+nCO_2$$

While it is known that syngas can be converted directly into light olefins in a relatively high yield employing a variety of catalyst compositions, such syntheses have been impractical or uneconomical due to problems associated with inadequate catalyst stability. The catalysts employed in the alkylation process of the present invention not only possess high levels of activity, but they also possess a degree of stability which far exceeds that of catalysts previously proposed for the direct conversion of syngas to light olefins.

The term "aromatic" is used herein in accordance with its recognized meaning in the art and includes substituted and unsubstituted mono- and polynuclear aromatic compounds. Compounds of an aromatic

character which possess a hetero atom are also useful provided they do not act as catalyst poisons. For example, thiophene can be alkylated in accordance with the process of this invention but pyridine, which is basic in character, would ordinarily not be considered a useful starting material due to its adverse effect on the catalysts used in the process, all of which are acidic in nature.

Substituted aromatic compounds which can be alkylated must possess at least one hydrogen atom directly bonded to the aromatic nucleus. With this proviso, the aromatic rings can be substituted with one or more halogen atoms and/or alkyl, aryl, alkaryl, hydroxy, alkoxy, aryloxy, cycloalkyl and/or other groups which do not interfere with the alkylation reaction.

Suitable aromatic hydrocarbons include benzene which is preferred, especially where the sole function of the aromatic compound is to serve as a temporary trapping device for alkyl groups in an overall synthesis for light olefins as described above; as well as naphthalene, anthracene, naphthacene, chrysene, pyrene, triphenylene, pentacene, picene, perylene, coronene and phenanthrene.

Generally, the alkyl groups which may be present as substituents on the aromatic compound contain from one to 22 carbon atoms and preferably from one to eight carbon atoms, most preferably from one to four carbon atoms.

Suitable alkyl substituted aromatic compounds therefore include toluene; xylene; isopropylbenzene; normal propylbenzene; alpha - methylnaphthalene; ethylbenzene; cumene; mesitylene; durene; p-cymene; butylbenzene; pseudocumene; o-diethylbenzene; m-diethylbenzene; p-diethylbenzene; isoamylbenzene; isohexylbenzene; pentaethylbenzene; pentamethylbenzene; 1,2,3,4 - tetraethylbenzene; 1,2,3,5 - tetramethylbenzene; 1,2,4 - triethylbenzene; 1,2,3 - trimethylbenzene; m-butyltoluene; p-butyltoluene; 3,5 - diethyltoluene; o-ethyltoluene; p-ethyltoluene; m-propyltoluene; 4 - ethyl - m - xylene; dimethylnaphthalenes; ethylnaphthalenes; 2,3 - dimethylanthracene; 9 - ethylanthracene; 2-methylanthracene; o-methylanthracene; 9,10 - dimethylphenanthrene; and 3 - methyl - phenanthrene. Higher molecular weight alkylaromatic hydrocarbons may also be used as starting materials and include aromatic hydrocarbons such as are produced by the alkylation of aromatic hydrocarbons with olefin polymers. Such products are frequently referred to in the art as alkylate, and include hexylbenzene, nonylbenzene, dodecylbenzene, pentadecylbenzene, hexyltoluene, nonyltoluene, dodecyltoluene and pentadecyltoluene. Very often alkylate is obtained as a high boiling fraction in which the alkyl group attached to the aromatic nucleus varies in size from about $C_6$ to about $C_{12}$.

Hydroxy substituted aromatic compounds will generally be the hydroxybenzenes, hydroxynaphthalenes and bis-phenols. Useful hydroxy-substituted aromatic compounds include phenol, o-cresol, m-cresol, p-cresol, catechol, resorcinol, hydroquinone, pyrogallol, alpha-naphthol, alpha-anthrol, and methylaniline and bisphenol-A.

Suitable halo-substituted aromatic compounds include chlorobenzene, o-dichlorobenzene, p-dichlorobenzene, bromobenzene, o-dibromobenzene, and p-dibromobenzene.

Suitable aromatic compounds substituted with aryl or alkaryl groups include diphenyl, triphenyl, diphenylmethane, triphenylmethane, dinaphthyl and stilbene.

Suitable alkoxy or aryloxy group-substituted compounds include anisole and phenetole.

Suitable cycloalkyl group-substituted compounds include phenylcyclohexane.

Suitable aromatic compounds carrying at least two different substituents include o-, m-, and p-chlorophenol, 2,5-dichlorophenol, thymol, m-ethylphenol, p-t-butylphenol, o-phenylphenol, p-phenylphenol, guaiacol, eugenol and isoeugenol.

Other aromatic compounds suitable for use in the process of this invention include phenyl acetate, indene and fluorene.

Some suitable aromatic compounds possessing hetero atoms include thiophene, furan, and quinoline.

The alkylating agent which is used in the process of this invention is a mixture of hydrogen and/or a hydrogen-containing substance which provides hydrogen under the process conditions, and a carbon oxide, that is carbon monoxide and/or carbon dioxide. Gaseous mixtures of hydrogen and carbon monoxide, of hydrogen and carbon dioxide and of hydrogen, carbon monoxide and carbon dioxide, which may be obtained from the gasification of a fossil fuel such as coal or natural gas and, when derived in this manner, are collectively referred to as synthesis gas or "syngas", are preferred alkylating agents in the process of the invention.

A typical, purified synthesis gas will have the composition, on a water-free basis, in volume percentages, as follows: hydrogen, 51; carbon monoxide, 40; carbon dioxide, 4; methane, 1; and nitrogen, 4.

The synthesis gas may be prepared from fossil fuels by any known method, including *in situ* gasification processes, for example the underground partial combustion of coal and petroleum deposits. The term fossil fuels is intended to include anthracite and bituminous coal, lignite, crude petroleum, shale oil, oil from tar sands, natural gas, as well as fuels derived from simple physical separations or more profound transformations of these materials, including coked coal, petroleum coke, gas oil, residua from petroleum distillation, and any two or more of the above materials in combination. Other carbonaceous fuels such as peat, wood and cellulosic waste materials also may be used.

Although various processes may be employed for the gasification, those of major importance depend either on the partial combustion of the fuel with an oxygen-containing gas or on the high temperature reaction of the fuel with steam, or on a combination of these two reactions.

The raw synthesis gas produced from fossil fuels will contain various impurities such as particulates, sulfur compounds, and metal carbonyl compounds, and will generally be characterized by a hydrogen-to-carbon oxides ratio which depends on the fossil fuel and the particular gasification technology utilized. In general, it is desirable for the efficiency of subsequent conversion steps to purify the raw synthesis gas by the removal of impurities. Techniques for such purification are well known.

Mixtures of carbon monoxide and a hydrogen-containing substance such as water which react under the conditions of the alkylation reaction to provide hydrogen, can also be used.

The volume ratio of hydrogen to carbon oxide in the alkylating agent is not critical and can vary widely. In general, however, it is preferred to adjust the hydrogen-to-carbon monoxide volume ratio to be within the range of from 0.2 to 10 and particularly in the range of from 0.4 to 3, upon contact with the catalyst. Should the alkylating agent be excessively rich in carbon oxides, its composition may be brought within the preferred range by the well known water-gas shift reaction. Purified synthesis gas having or adjusted to contain the desired volume ratio of hydrogen-to-carbon monoxide within the range of from 0.2 to 10 will be referred to as adjusted synthesis gas.

The heterogeneous catalyst of this invention comprises a metal or metal-containing component selected from iron, cobalt, ruthenium, manganese, rhodium and osmium, and a zeolite having a Constraint Index of 12 or less. Iron, cobalt and ruthenium are preferred, iron and cobalt for their relatively low cost and ruthenium, while expensive, for its comparatively high level of activity.

The weight ratio of metal, calculated upon the basis of the metal in its free, uncombined state, to zeolite can range from 0.3 to 3. In the case of the more active metals such as ruthenium, this ratio can vary from 0.001 to 1 and preferably from 0.003 to 0.1. The catalyst can be used in the form of a loose mixture or as pellets or an extrudate.

The metal or metal-containing component can be incorporated by a variety of methods. For example:

(1) The metal or metal-containing component, preferably the oxide, is simply mixed or ground together with the zeolite.

(2) The metal in the form of a compound dissolved in a suitable solvent is impregnated onto an inert support such as activated carbon, silica, alumina, titania, magnesia, zirconia or a clay, and following calcination to the oxide, the supported metal oxide is mixed with the zeolite.

(3) A solution of a compound of the metal in a suitable solvent is impregnated directly onto the zeolite followed by calcination of the compound to the oxide.

(4) The metal or metal-containing component is incorporated onto the zeolite during the crystallization step of manufacturing the zeolite.

(5) The metal or metal-containing component is incorporated onto the zeolite by an ion exchange process.

(6) The metal or metal-containing component present as an intercalate in graphite is mixed with the zeolite.

When a metal compound other than an oxide is used, the compound can be converted to the oxide by any of the conventional techniques of thermal decomposition (calcination) whose operational parameters are well known.

For the impregnation step employed in methods (2) and (3), there may be used metal salts and other compounds, for example halides, nitrates, oxalates, carbonates, acetates and acetylacetonates. Aqueous or non-aqueous solvents, for example methanol, acetone, chloroform, hexane and benzene, can be used depending on the solubility of the metal compound selected.

After impregnation, the catalyst is vacuum dried at 100°C and then reduced at a temperature ranging from 150°C to 600°C, and preferably from 250°C to 500°C, with a reducing gas such as hydrogen or a hydrogen-containing gas such as an $H_2$/CO mixture, to activate the catalyst. Activation can be carried out under pressure. However, when an $H_2$/CO mixture is used, low pressure such as atmospheric pressure is preferred. The optimum time and temperature to accomplish activation depends on the particular compound used and can be determined experimentally for a given metal compound employing routine experimentation. It is, of course, equally possible to effect activation *in situ* in the alkylation zone.

The catalysts can also be combined with one or more promoters, this being especially desirable when iron and/or cobalt are present. Suitable promoters include such metal and metal-containing components as the alkali metals, particularly potassium, the alkaline earth metals, and copper, the compounds of these metals, particularly the carbonates, and their mixtures. Typically, the promoters are added in fairly small quantities, for example up to 5% by weight of the metal component of the non-promoted catalyst.

The zeolites used in the catalysts are natural or synthetic porous tectosilicates characterized by a rigid crystalline framework structure composed of an assembly of silicon atoms and at least a trace amount of a trivalent metal atom, preferably aluminum, but which can also be iron, boron, gallium or chromium for example, or mixtures thereof, the silicon atoms and trivalent metal atoms each being surrounded by a tetrahedron of shared oxygen atoms, and having a precisely defined pore structure. Exchangeable cations are present in the pores.

The preferred zeolites are selected from the natural and synthetic crystalline aluminosilicates. These zeolites are capable of inducing profound transformations of aliphatic hydrocarbons to aromatic hydrocarbons in commercially desirable yields and are generally highly effective in alkylation,

isomerization, disproportionation and other reactions involving aromatic hydrocarbons. Although they have unusually low alumina contents, i.e., high silica to alumina ratios, they are very active even with silica to alumina ratios exceeding 30 and exhibit activity even with very high silica to alumina ratios, for example 10,000 and even higher. This activity is surprising since catalytic activity of zeolites is generally attributed to framework aluminum atoms and cations associated with these aluminum atoms. These zeolites retain their crystallinity for long periods in spite of the presence of steam even at high temperatures which induce irreversible collapse of the crystal framework of other zeolites, for example of the X and A type. Furthermore, carbonaceous deposits, when formed, may be removed by burning at higher than usual temperatures to restore activity. In many environments the zeolites of this class exhibit very low coke forming capability, conducive to very long times on stream between burning regenerations.

An important characteristic of the crystal structure of this class of zeolites is that it provides constrained access to, and egress from, the intra-crystalline free space by virtue of having a pore dimension greater than about 5 Angstroms and pore windows of about a size such as would be provided by 10-membered rings of oxygen atoms. It is to be understood, of course, that these rings are those formed by the regular disposition of the tetrahedra making up the anionic framework of the crystalline aluminosilicate, the oxygen atoms themselves being bonded to the silicon or aluminum atoms at the centers of the tetrahedra. Briefly, the preferred zeolites useful in this invention possess, in combination: a silica to alumina ratio of at least about 12; and a structure providing constrained access to the crystalline free space.

The silica to alumina ratio referred to may be determined by conventional analysis. This ratio is meant to represent, as closely as possible, the ratio in the rigid anionic framework of the zeolite crystal and to exclude aluminum in the binder or in cationic or other form within the channels. Although zeolites with a silica to alumina ratio of at least 12 are useful, it is preferred to use zeolites having higher ratios of at least about 30. Such zeolites, after activation, acquire an intracrystalline sorption capacity for normal hexane which is greater than that for water, i.e., they exhibit "hydrophobic" properties. It is believed that this hydrophobic character is advantageous in the present invention.

The zeolites useful as catalysts in this invention freely sorb normal hexane and have a pore dimension greater than about 5 Angstroms. In addition, their structure must provide constrained access to some larger molecules. It is sometimes possible to judge from a known crystal structure whether such constrained access exists. For example, if the only pore windows in a crystal are formed by 8-membered rings of oxygen atoms, then access by molecules of larger cross-section than normal hexane is substantially excluded and the zeolite is not of the desired type. Zeolites with windows of 10-membered rings are preferred, although excessive puckering or pore blockage may render these zeolites substantially ineffective.

Rather than attempt to judge from crystal structure whether or not a zeolite possesses the necessary constrained access, a simple determination of the "Constraint Index" may be made by continuously passing a mixture of equal weight of normal hexane and 3-methylpentane over a small sample, approximately 1 gram or less, of zeolite at atmospheric pressure according to the following procedure. A sample of the zeolite, in the form of pellets or extrudate, is crushed to a particle size about that of coarse sand and mounted in a glass tube. Prior to testing, the zeolite is treated with a stream of air at 538°C for at least 15 minutes. The zeolite is then flushed with helium and the temperature adjusted to between 288°C and 510°C to give an overall 1 conversion between 10% and 60%. The mixture of hydrocarbons is passed at 1 liquid hourly space velocity (1 volume of liquid hydrocarbon per volume of catalyst per hour) over the zeolite with a helium dilution to give a helium to total hydrocarbon mole ratio of 4:1. After 20 minutes on stream, a sample of the effluent is taken and analyzed, most conveniently by gas chromatography, to determine the fraction remaining unchanged for each of the two hydrocarbons.

The "constraint index" is calculated as follows:

$$\text{Constraint Index} = \frac{\log_{10}\ (\text{fraction of n-hexane remaining})}{\log_{10}\ (\text{fraction of 3-methylpentane remaining})}$$

The Constraint Index approximates the ratio of the cracking rate constants for the two hydrocarbons. Catalysts suitable for the present invention are those which employ a zeolite having a Constraint Index of 12.0 or less. Constraint Index (CI) values for some typical zeolites included within the scope of this invention are:

| Zeolite | C.I. |
|---|---|
| ZSM-5 | 8.3 |
| ZSM-11 | 8.7 |
| ZSM-35 | 6.0 |
| TMA Offretite | 3.7 |
| ZSM-38 | 2.0 |
| ZSM-12 | 2 |
| Beta | 0.6 |
| ZSM-4 | 0.5 |
| Acid mordenite | 0.5 |
| REY | 0.4 |

Zeolites with a Constraint Index greater than 12 such as erionite (CI=38) are not useful for the purposes of this invention. In the practice of the present invention, zeolites having Constraint Index values above about 2 up to the maximum value of 12 are preferred for the production of alkylated aromatics in which the alkyl side changes are of relatively short length, for example from 2 to about 8 carbon atoms, and zeolites having Constraint Index values of 2 and less are preferred for the production of alkylated aromatics possessing relatively long side chains, for example from about 9 to about 22 carbon atoms. Thus, for example, zeolites such as those of the ZSM-5, ZSM-11 and ZSM-35 type are preferred for making short alkyl chain aromatics and those of the ZSM-4, ZSM-12 and ZSM-38 type are preferred for making long alkyl chain aromatics.

The Constraint Index is an important, and even critical, definition of those zeolites which are useful to catalyze the instant process. The very nature of this parameter and the recited technique by which it is determined, however, admit of the possibility that a given zeolite can be tested under somewhat different conditions and thereby have different Constraint Indexes. Constraint Index seems to vary somewhat with severity of operation (conversion). Therefore, it will be appreciated that it may be possible to so select test conditions to establish multiple Constraint Indexes for a particular given zeolite which may be both inside and outside the above defined range of 12 and less.

Thus, it should be understood that the parameter and property "Constraint Index" as such value is used herein is an inclusive rather than an exclusive value. That is, a zeolite when tested by any combination of conditions within the testing definition set forth herein above to have a Constraint Index of 12 and less is intended to be included in the definition regardless that the same zeolite tested under other conditions may give a Constraint Index value above 12.

The preferred zeolites for the purposes of the invention are ZSM-5, ZSM-11, ZSM-12, ZSM-21, and other similar materials. ZSM-5 is especially preferred.

ZSM-5 is described in U.S. Patent 3,702,886; ZMS-11 is described in U.S. Patent 3,709,979; and ZSM-12 is described in U.S. Patent 3,832,449.

Evidence has been adduced which suggests that ZSM-21 may be composed of at least two different zeolites designated ZSM-35 and ZSM-38, one or both of which are the effective material insofar as the catalysis of this invention is concerned. ZSM-35 is described in U.S. Patent 4,016,245 and ZSM-38 is described in U.S. Patent 4,046,859.

The specific zeolites described, when prepared in the presence of organic cations, are substantially catalytically inactive, possibly because the intracrystalline free space is occupied by organic cations from the forming solution. They may be activated by heating in an inert atmosphere at 538°C for one hour, for example, followed by base exchange with ammonium salts followed by calcination at 538°C in air. The presence of organic cations in the forming solution may not be absolutely essential to the formation of this type of zeolite. More generally, it is desirable to activate this type zeolite by base exchange with ammonium salts followed by calcination in air at 538°C for from 15 minutes to 24 hours.

Natural zeolites may sometimes be converted to this type of zeolite of various activation procedures and other treatments such as base exchange, steaming, alumina extraction and calcination, alone or in combination. Natural minerals which may be so treated include ferrierite, brewsterite, stilbite, dachiardite, epistilbite, heulandite and clinoptilolite.

The zeolites used as catalysts in this invention may be in the hydrogen form or they may be base

exchanged or impregnated to contain ammonium or a metal cation complement. It is desirable to calcine the zeolite after base exchange. The metal cations that may be present include any of the cations of the metals of Groups I through VIII of the periodic table. However, in the case of Group IA metals, the cation content should in no case be so large as to substantially eliminate the activity of the zeolite for the catalysis being employed in the invention.

In a preferred aspect of this invention, the zeolites useful as components of the catalysts are those having a crystal framework density, in the dry hydrogen form, of not substantially below 1.6 grams per cubic centimeter. Therefore, the preferred catalysts of this invention are those comprising a zeolite having a Constraint Index as defined above of 12 or less, a silica to alumina ratio of at least 12 and a dried crystal density of not substantially less than 1.6 grams per cubic centimeter. The dry density for known structures may be calculated from the number of silicon plus aluminum atoms per 1000 cubic Angstroms, as given, for example on page 19 of the article on Zeolite Structure by W. M. Meier, included in "Proceedings of the Conference on Molecular Sieves, London, April 1967", published by the Society of Chemical Industry, London, 1968. When the crystal structure is unknown, the crystal framework density may be determined by classical pyknometer techniques. For example, it may be determined by immersing the dry hydrogen form of the zeolite in an organic solvent which is not sorbed by the crystal. It is possible that the unusual sustained activity and stability of this class of zeolites is associated with a relatively small amount of free space within the crystal, which might be expected to result in more stable structures. This free space, however, seems to be important as the locus of catalytic activity.

Crystal framework densities of some typical zeolites are:

| Zeolite | Void volume | Framework density |
|---|---|---|
| Ferrierite | 0.28 cc/cc | 1.76 g/cc |
| Mordenite | .28 | 1.7 |
| ZSM-5, -11 | .29 | 1.79 |
| Dachiardite | .32 | 1.72 |
| L | .32 | 1.61 |
| Clinoptilolite | .34 | 1.71 |
| Laumontite | .34 | 1.77 |
| ZSM-4 (Omega) | .38 | 1.65 |
| Heulandite | .39 | 1.69 |
| P | .41 | 1.57 |
| Offretite | .40 | 1.55 |
| Levynite | .40 | 1.54 |
| Gmelinite | .44 | 1.46 |
| Chabazite | .47 | 1.45 |
| A | .5 | 1.3 |
| Y | .48 | 1.27 |

In the process of the present invention, the starting aromatic compound(s) and the alkylating agent are contacted with the catalyst in an alkylation zone maintained at elevated temperature, for example from 180 to 450°C, and preferably from 210 to 400°C. Pressure within the alkylation zone can vary widely and pressures of the order of from atmospheric to 10,000 kPa, advantageously from 600 to 6,000 kPa generally provide good results. The amount of catalyst required can also vary and for practical rates of conversion, will ordinarily be sufficient to provide a gas hourly space velocity (GHSV) at standard temperature and pressure (STP) of from 30 to 10,000 and preferably from 100 to 3,000. It is preferred to use a stoichiometric excess of aromatic compound(s) compared to the carbon oxide(s) content of the alkylating agent in order to

ensure maximum consumption of the latter. Preferred mole ratios of aromatic compound(s) to carbon oxide(s) are from 0.1 to 20, more preferably from 0.2 to 5.

The heterogeneous catalyst can be used as a fixed bed or a fluidized bed, or a liquid slurry reactor may be used. The product stream containing the alkyl aromatic mixture, unreacted gases and steam can be cooled and the hydrocarbons recovered by any known technique. The recovered alkyl aromatics can be further separated by distillation or other means to provide relatively pure alkyl aromatics.

The following Example illustrates the invention.

Example

Benzene was reacted with syngas under the conditions and with the results indicated below.

| | |
|---|---|
| Temperature, °C | 315 |
| Pressure, kPa | 2533 |
| Catalyst | Fe-K-Cu*/HZSM-5 |
| Volume ratio of hydrogen to carbon monoxide | 0.68 |
| Mole ratio of benzene to carbon monoxide | 0.6 |
| GHSV | 630 |
| Total syngas conversion, mole % | 97 |
| Trapping efficiency**, % | 58 |
| Alkylaromatics distribution wt. % | |
| Ethylbenzene | 6 |
| Propylbenzenes | 47 |
| Butylbenzenes | 25 |
| Pentylbenzenes | 12 |
| Hexylbenzene | 5 |
| Other (methyl aromatics) | 5 |
| | 100 |

* Fe 65%; K 0.31%; Cu 0.31%, coprecipitated.

** Percentage of hydrocarbons produced from syngas but subsequently converted to the alkyl groups of alkylaromatics by reacting with benzene.

As described above, the alkylaromatic product stream resulting from the process of the invention, with or without being separated into its individual components or a plurality of fractions, can be subjected to dealkylation in accordance with known and conventional procedures, to provide olefin. The dealkylated aromatic can thereafter be recycled to the alkylation zone to trap additional quantities of hydrocarbons in the form of alkyl groups. In general, dealkylation may be carried out employing acidic catalysts, for example silica, silica-alumina, magnesia and zeolites, at a temperature of from about 400 to about 650°C, preferably from about 450 to about 600°C, and a pressure of from about 100 to about 2,000 kPa, preferably from about 200 to about 1,000 kPa. Hydrogen is preferably present in the dealkylation zone in order to increase catalyst stability.

**Claims**

1. A process for alkylating an aromatic compound which comprises reacting at least one aromatic compound having at least one hydrogen atom directly bonded to the aromatic nucleus with an alkylating agent comprising a mixture of hydrogen and/or a hydrogen-containing substance which provides hydrogen under the process conditions, and a carbon oxide, at an elevated temperature in the presence of a catalyst, characterized in that the catalyst comprises a metal or metal-containing component selected from iron, cobalt, ruthenium, manganese, rhodium and osmium, and a zeolite having a Constraint Index of

not more than 12, and recovering a product comprising an aromatic compound having at least one more alkyl group of at least two carbon atoms than the starting aromatic compound.

2. A process according to claim 1, wherein the starting aromatic compound is benzene.

3. A process according to claim 1 or claim 2, wherein the alkylating agent is a gaseous mixture of hydrogen and carbon monoxide.

4. A process according to any one of claims 1 to 3, wherein the metal or metal-containing component is iron, cobalt or ruthenium.

5. A process according to any one of claims 1 to 4, wherein the zeolite is ZSM-5.

6. A process according to any one of claims 1 to 5, wherein the product aromatic compound is subjected to dealkylation to provide olefin and the starting aromatic compound, the latter being separated from the olefin and recycled to the alkylation reaction.

**Patentansprüche**

1. Verfahren zur Alkylierung einer aromatischen Verbindung, das die Umsetzung wenigstens einer aromatischen Verbindung mit wenigstens einem Wasserstoffatom, das direkt an den aromatischen Kern gebunden ist, mit einem Alkylierungsmittel, das eine Mischung von Wasserstoff und/oder einer wasserstoffhaltigen Substanz, die unter den Verfahrensbedingungen Wasserstoff liefert, mit einem Kohlenoxid umfaßt, bei einer erhöhten Temperatur in Gegenwart eines Katalysators umfaßt, dadurch gekennzeichnet, daß der Katalysator ein Metall oder einen metallhaltigen Bestandteil, die aus Eisen, Kobalt, Ruthenium, Mangan, Rhodium und Osmium ausgewählt sind, sowie einen Zeolithen mit einem Grenzwertindex (constraint index) von nicht mehr als 12 umfaßt, sowie Gewinnen eines Produkts, das eine aromatische Verbindung umfaßt, die wenigstens eine Alkylgruppe mit wenigstens zwei Kohlenstoffen mehr aufweist, als die aromatische Ausgangsverbindung.

2. Verfahren nach Anspruch 1, bei dem die aromatische Ausgangsverbindung Benzol ist.

3. Verfahren nach Anspruch 1 oder Anspruch 2, bei dem das Alkylierungsmittel eine gasförmige Mischung von Wasserstoff und Kohlenmonoxid ist.

4. Verfahren nach einem der Ansprüche 1 bis 3, bei dem das Metall oder der metallhaltige Bestandteil Eisen, Kobalt oder Ruthenium ist.

5. Verfahren nach einem der Ansprüche 1 bis 4, bei dem der Zeolith ZSM-5 ist.

6. Verfahren nach einem der Ansprüche 1 bis 5, bei dem die aromatische Produktverbindung einer Entalkylierung unterzogen wird, um ein Olefin und aromatische Ausgangsverbindung zu erzeugen, wobei die letztere von dem Olefin abgetrennt und in die Alkylierungsreaktion zurückgeführt wird.

**Revendications**

1. Un procédé d'alkylation d'un composé aromatique qui comprend la réaction d'au moins un composé aromatique ayant au moins un atome d'hydrogène fixé directement au noyau aromatique avec un agent d'alkylation comprenant un mélange d'hydrogène et/ou d'une substance contenant de l'hydrogène qui fournit de l'hydrogène dans les conditions opératoires, et un oxyde de carbone, à une température élevée en présence d'un catalyseur, caractérisé en ce que le catalyseur comprend un métal ou un composant contenant un métal choisi parmi le fer, le cobalt, le ruthénium, le manganèse, le rhodium et l'osmium et une zéolite ayant un indice de contrainte ne dépassant pas 12 et la récupération d'un produit comprenant un composé aromatique ayant au moins un groupe alkyle d'au moins deux atomes de carbone de plus que le composé aromatique de départ.

2. Un procédé selon la revendication 1, dans lequel le composé aromatique de départ est le benzène.

3. Un procédé selon la revendication 1 ou la revendication 2, dans lequel l'agent d'alkylation est un mélange gazeux d'hydrogène et de monoxyde de carbone.

4. Un procédé selon l'une quelconque des revendications 1 à 3, dans lequel le métal ou le composant contenant un métal est le fer, le cobalt ou le ruthénium.

5. Un procédé selon l'une quelconque des revendications 1 à 4, dans lequel la zéolite est la ZSM-5.

6. Un procédé selon l'une quelconque des revendications 1 à 5, dans lequel le composé aromatique produit est soumis à une désalkylation pour fournir une oléfine et le composé aromatique de départ, ce dernier étant séparé de l'oléfine et recyclé dans la réaction d'alkylation.